# EUROPEAN PATENT APPLICATION

(11) **EP 2 848 628 A1**
(43) Date of publication of application: **18.03.2015**
(21) Application number: 14194189.8
(22) Date of filing: 24.01.2011
(51) Int. Cl.: C07K 7/00, A61K 38/08, C07K 5/11, A61K 38/07, A61P 39/06

(54) **Aromatic-cationic peptides and uses of same**

(30) Priority: 25.01.2010 US 298062 P
(62) Divisional of application: 14155476.6
(71) Applicant: Cornell University, Ithaca, NY 14850 (US); Institut de Recherches Cliniques de Montreal, Montreal, Quebec H2W 1R7 (CA)
(72) Inventor: Szeto, Hazel H, New York, NY 10128 (US); Schiller, Peter W, Montreal, Québec H2W 1R7 (CA)
(74) Representative: Atkinson, Jennifer

(57) **Abstract**

The disclosure provides aromatic-cationic peptide compositions and methods of preventing or treating disease using the same. The methods comprise administering to the subject an effective amount of an aromatic-cationic peptide to subjects in need thereof.

## Description

### CROSS-REFERENCE TO RELATED APPLICATIONS

This application claims priority to U.S. Provisional Patent Application No. 61/298,062, filed January 25, 2010, the entire contents of which is hereby incorporated by reference in its entirety.

### TECHNICAL FIELD

The present technology relates generally aromatic-cationic peptide compositions and methods of preventing or treating disease using the same.

### SUMMARY

In one aspect, the present technology provides an aromatic-cationic peptide or a pharmaceutically acceptable salt thereof. In some embodiments, the peptide is selected from the group consisting of:
D-Arg-Dmt-Lys-Trp-NH₂;
D-Arg-Trp-Lys-Trp-NH₂;
D-Arg-Dmt-Lys-Phe-Met-NH₂;
H-D-Arg-Dmt-Lys(*N^{α}*Me)-Phe-NH₂;
H-D-Arg-Dmt-Lys-Phe(*N*Me)-NH₂;
H-D-Arg-Dmt-Lys(*N^{α}*Me)-Phe(*N*Me)-NH₂;
H-D-Arg(*N^{α}*Me)-Dmt(*N*Me)-Lys(*N^{α}*Me)-Phe(*N*Me)-NH₂;
D-Arg-Dmt-Lys-Phe-Lys-Trp-NH₂;
D-Arg-Dmt-Lys-Dmt-Lys-Trp-NH₂;
D-Arg-Dmt-Lys-Phe-Lys-Met-NH₂;
D-Arg-Dmt-Lys-Dmt-Lys-Met-NH₂;
H-D-Arg-Dmt-Lys-Phe-Sar-Gly-Cys-NH₂;
H-D-Arg-Ψ[CH₂-NH]Dmt-Lys-Phe-NH₂;
H-D-Arg-Dmt-Ψ[CH₂-NH]Lys-Phe-NH₂;
H-D-Arg-Dmt-LysΨ[CH₂-NH]Phe-NH₂; and
H-D-Arg-Dmt-Ψ[CH₂-NH]Lys-Ψ[CH₂-NH]Phe-NH₂.

In some embodiments, "Dmt" refers to 2',6'-dimethyltyrosine (2'6'-Dmt) or 3',5'-dimethyltyrosine (3'5'Dmt).

In another aspect, the disclosure provides a pharmaceutical composition comprising the aromatic cationic peptide and a pharmaceutically acceptable carrier.

In another aspect, the disclosure provides a method for reducing oxidative damage in a mammal in need thereof, the method comprising administering to the mammal an effective amount of one or more aromatic cationic peptides.

In another aspect, the disclosure provides a method for reducing the number of mitochondria undergoing mitochondrial permeability transitioning (MPT), or preventing mitochondrial permeability transitioning in a mammal in need thereof, the method comprising administering to the mammal an effective amount of one or more aromatic cationic peptides.

### BRIEF DESCRIPTION OF THE FIGURES

FIG. 1 is a chart showing that the peptide D-Arg-Dmt-Lys-Phe-NH₂ increases the rate of cytochrome c (cyt c) reduction. Reduced cyt c was measured by absorbance at 550 nm. The peptide dose-dependently increased the rate of cyt c reduction induced by 40 µM NAC. The peptide alone at 100 µM had no effect.
FIG. 2 is a chart showing treatment with D-Arg-Dmt-Lys-Phe-NH₂ increased state 3 respiration in isolated renal mitochondria after 20 min IR injury (p<0.01).
FIG. 3 is a chart showing that treatment with D-Arg-Dmt-Lys-Phe-NH₂ increased ATP content in rat kidney at 1 h after IR injury (P<0.05).
FIG. 4 is a chart showing that H-Phe-D-Arg-Phe-Lys-Cys-NH₂ maintains redox status in rat kidney after ischemia reperfusion (IR).

### DETAILED DESCRIPTION

It is to be appreciated that certain aspects, modes, embodiments, variations and features of the invention are described below in various levels of detail in order to provide a substantial understanding of the present invention. The definitions of certain terms as used in this specification are provided below. Unless defined otherwise, all technical and scientific terms used herein generally have the same meaning as commonly understood by one of ordinary skill in the art to which this invention belongs.

As used in this specification and the appended claims, the singular forms "a", "an" and "the" include plural referents unless the content clearly dictates otherwise. For example, reference to "a cell" includes a combination of two or more cells, and the like.

As used herein, the "administration" of an agent, drug, or peptide to a subject includes any route of introducing or delivering to a subject a compound to perform its intended function. Administration can be carried out by any suitable route, including orally, intranasally, parenterally (intravenously, intramuscularly, intraperitoneally, or subcutaneously), or topically. Administration includes self-administration and the administration by another.

As used herein, the term "amino acid" includes naturally-occurring amino acids and synthetic amino acids, as well as amino acid analogs and amino acid mimetics that function in a manner similar to the naturally-occurring amino acids. Naturally-occurring amino acids are those encoded by the genetic code, as well as those amino acids that are later modified, *e.g*., hydroxyproline, γ-carboxyglutamate, and O-phosphoserine. Amino acid analogs refers to compounds that have the same basic chemical structure as a naturally-occurring amino acid, *i.e.,* an α-carbon that is bound to a hydrogen, a carboxyl group, an amino group, and an R group, *e.g*., homoserine, norleucine, methionine sulfoxide, methionine methyl sulfonium. Such analogs have modified R groups (e.g., norleucine) or modified peptide backbones, but retain the same basic chemical structure as a naturally-occurring amino acid. Amino acid mimetics refers to chemical compounds that have a structure that is different from the general chemical structure of an amino acid, but that functions in a manner similar to a naturally-occurring amino acid. Amino acids can be referred to herein by either their commonly known three letter symbols or by the one-letter symbols recommended by the IUPAC-IUB Biochemical Nomenclature Commission.

As used herein, the term "effective amount" refers to a quantity sufficient to achieve a desired therapeutic and/or prophylactic effect. In the context of therapeutic or prophylactic applications, the amount of a composition administered to the subject will depend on the type and severity of the disease and on the characteristics of the individual, such as general health, age, sex, body weight and tolerance to drugs. It will also depend on the degree, severity and type of disease. The skilled artisan will be able to determine appropriate dosages depending on these and other factors. The compositions can also be administered in combination with one or more additional therapeutic compounds.

An "isolated" or "purified" polypeptide or peptide is substantially free of cellular material or other contaminating polypeptides from the cell or tissue source from which the agent is derived, or substantially free from chemical precursors or other chemicals when chemically synthesized. For example, an isolated aromatic-cationic peptide would be free of materials that would interfere with diagnostic or therapeutic uses of the agent. Such interfering materials may include enzymes, hormones and other proteinaceous and nonproteinaceous solutes.

As used herein, the terms "polypeptide", "peptide", and "protein" are used interchangeably herein to mean a polymer comprising two or more amino acids joined to each other by peptide bonds or modified peptide bonds, *i.e.,* peptide isosteres. Polypeptide refers to both short chains, commonly referred to as peptides, glycopeptides or oligomers, and to longer chains, generally referred to as proteins. Polypeptides may contain amino acids other than the 20 gene-encoded amino acids. Polypeptides include amino acid sequences modified either by natural processes, such as post-translational processing, or by chemical modification techniques that are well known in the art.

As used herein, the terms "treating" or "treatment" or "alleviation" refers to both therapeutic treatment and prophylactic or preventative measures, wherein the object is to prevent or slow down (lessen) the targeted pathologic condition or disorder. It is also to be appreciated that the various modes of treatment or prevention of medical conditions as described are intended to mean "substantial", which includes total but also less than total treatment or prevention, and wherein some biologically or medically relevant result is achieved.

As used herein, "prevention" or "preventing" of a disorder or condition refers to a compound that reduces the occurrence of the disorder or condition in the treated sample relative to an untreated control sample, or delays the onset or reduces the severity of one or more symptoms of the disorder or condition relative to the untreated control sample.

The present technology relates to the treatment or prevention of disease by administration of certain aromatic-cationic peptides.

The aromatic-cationic peptides are water-soluble and highly polar. Despite these properties, the peptides can readily penetrate cell membranes. The aromatic-cationic peptides typically include a minimum of three amino acids or a minimum of four amino acids, covalently joined by peptide bonds. The maximum number of amino acids present in the aromatic-cationic peptides is about twenty amino acids covalently joined by peptide bonds. Suitably, the maximum number of amino acids is about twelve, about nine, or about six.

The amino acids of the aromatic-cationic peptides can be any amino acid. As used herein, the term "amino acid" is used to refer to any organic molecule that contains at least one amino group and at least one carboxyl group. Typically, at least one amino group is at the α position relative to a carboxyl group. The amino acids may be naturally occurring. Naturally occurring amino acids include, for example, the twenty most common levorotatory (L) amino acids normally found in mammalian proteins, *i.e.,* alanine (Ala), arginine (Arg), asparagine (Asn), aspartic acid (Asp), cysteine (Cys), glutamine (Gln), glutamic acid (Glu), glycine (Gly), histidine (His), isoleucine (Ile), leucine (Leu), lysine (Lys), methionine (Met), phenylalanine (Phe), proline (Pro), serine (Ser), threonine (Thr), tryptophan, (Trp), tyrosine (Tyr), and valine (Val). Other naturally occurring amino acids include, for example, amino acids that are synthesized in metabolic processes not associated with protein synthesis. For example, the amino acids ornithine and citrulline are synthesized in mammalian metabolism during the production of urea. Another example of a naturally occurring amino acid includes hydroxyproline (Hyp).

The peptides optionally contain one or more non-naturally occurring amino acids. Optimally, the peptide has no amino acids that are naturally occurring. The non-naturally occurring amino acids may be levorotary (L-), dextrorotatory (D-), or mixtures thereof. Non-naturally occurring amino acids are those amino acids that typically are not synthesized in normal metabolic processes in living organisms, and do not naturally occur in proteins. In addition, the non-naturally occurring amino acids suitably are also not recognized by common proteases. The non-naturally occurring amino acid can be present at any position in the peptide. For example, the non-naturally occurring amino acid can be at the N-terminus, the C-terminus, or at any position between the N-terminus and the C-terminus.

The non-natural amino acids may, for example, comprise alkyl, aryl, or alkylaryl groups not found in natural amino acids. Some examples of non-natural alkyl amino acids include α-aminobutyric acid, β-aminobutyric acid, γ-aminobutyric acid, δ-aminovaleric acid, and ε-aminocaproic acid. Some examples of non-natural aryl amino acids include ortho-, meta, and para-aminobenzoic acid. Some examples of non-natural alkylaryl amino acids include ortho-, meta-, and para-aminophenylacetic acid, and γ-phenyl-β-aminobutyric acid. Non-naturally occurring amino acids include derivatives of naturally occurring amino acids. The derivatives of naturally occurring amino acids may, for example, include the addition of one or more chemical groups to the naturally occurring amino acid.

For example, one or more chemical groups can be added to one or more of the 2', 3', 4', 5', or 6' position of the aromatic ring of a phenylalanine or tyrosine residue, or the 4', 5', 6', or 7' position of the benzo ring of a tryptophan residue. The group can be any chemical group that can be added to an aromatic ring. Some examples of such groups include branched or unbranched C₁-C₄ alkyl, such as methyl, ethyl, n-propyl, isopropyl, butyl, isobutyl, or t-butyl, C₁-C₄ alkyloxy (*i.e.,* alkoxy), amino, C₁-C₄ alkylamino and C₁-C₄ dialkylamino *(e.g.,* methylamino, dimethylamino), nitro, hydroxyl, halo (*i.e.,* fluoro, chloro, bromo, or iodo). Some specific examples of non-naturally occurring derivatives of naturally occurring amino acids include norvaline (Nva) and norleucine (Nle).

Another example of a modification of an amino acid in a peptide is the derivatization of a carboxyl group of an aspartic acid or a glutamic acid residue of the peptide. One example of derivatization is amidation with ammonia or with a primary or secondary amine, e.g. methylamine, ethylamine, dimethylamine or diethylamine. Another example of derivatization includes esterification with, for example, methyl or ethyl alcohol. Another such modification includes derivatization of an amino group of a lysine, arginine, or histidine residue. For example, such amino groups can be acylated. Some suitable acyl groups include, for example, a benzoyl group or an alkanoyl group comprising any of the C₁-C₄ alkyl groups mentioned above, such as an acetyl or propionyl group.

The non-naturally occurring amino acids are suitably resistant or insensitive, to common proteases. Examples of non-naturally occurring amino acids that are resistant or insensitive to proteases include the dextrorotatory (D-) form of any of the above-mentioned naturally occurring L-amino acids, as well as L- and/or D- non-naturally occurring amino acids. The D-amino acids do not normally occur in proteins, although they are found in certain peptide antibiotics that are synthesized by means other than the normal ribosomal protein synthetic machinery of the cell. As used herein, the D-amino acids are considered to be non-naturally occurring amino acids.

In order to minimize protease sensitivity, the peptides should have less than five, less than four, less than three, or less than two contiguous L-amino acids recognized by common proteases, irrespective of whether the amino acids are naturally or non-naturally occurring. In one embodiment, the peptide has only D-amino acids, and no L-amino acids. If the peptide contains protease sensitive sequences of amino acids, at least one of the amino acids is preferably a non-naturally-occurring D-amino acid, thereby conferring protease resistance. An example of a protease sensitive sequence includes two or more contiguous basic amino acids that are readily cleaved by common proteases, such as endopeptidases and trypsin. Examples of basic amino acids include arginine, lysine and histidine.

The aromatic-cationic peptides should have a minimum number of net positive charges at physiological pH in comparison to the total number of amino acid residues in the peptide. The minimum number of net positive charges at physiological pH will be referred to below as (pₘ). The total number of amino acid residues in the peptide will be referred to below as (r). The minimum number of net positive charges discussed below are all at physiological pH. The term "physiological pH" as used herein refers to the normal pH in the cells of the tissues and organs of the mammalian body. For instance, the physiological pH of a human is normally approximately 7.4, but normal physiological pH in mammals may be any pH from about 7.0 to about 7.8.

"Net charge" as used herein refers to the balance of the number of positive charges and the number of negative charges carried by the amino acids present in the peptide. In this specification, it is understood that net charges are measured at physiological pH. The naturally occurring amino acids that are positively charged at physiological pH include L-lysine, L-arginine, and L-histidine. The naturally occurring amino acids that are negatively charged at physiological pH include L-aspartic acid and L-glutamic acid.

Typically, a peptide has a positively charged N-terminal amino group and a negatively charged C-terminal carboxyl group. The charges cancel each other out at physiological pH. As an example of calculating net charge, the peptide Tyr-Arg-Phe-Lys-Glu-His-Trp-D-Arg has one negatively charged amino acid (*i.e.,* Glu) and four positively charged amino acids (*i.e.,* two Arg residues, one Lys, and one His). Therefore, the above peptide has a net positive charge of three.

In one embodiment, the aromatic-cationic peptides have a relationship between the minimum number of net positive charges at physiological pH (pₘ) and the total number of amino acid residues (r) wherein 3pₘ is the largest number that is less than or equal to r + 1. In this embodiment, the relationship between the minimum number of net positive charges (pₘ) and the total number of amino acid residues (r) is as follows:

**TABLE 1. Amino acid number and net positive charges (3pₘ ≤ p+1)**

| **(r)** | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|
| **(pₘ)** | 1 | 1 | 2 | 2 | 2 | 3 | 3 | 3 | 4 | 4 | 4 | 5 | 5 | 5 | 6 | 6 | 6 | 7 |

In another embodiment, the aromatic-cationic peptides have a relationship between the minimum number of net positive charges (pₘ) and the total number of amino acid residues (r) wherein 2pₘ is the largest number that is less than or equal to r + 1. In this embodiment, the relationship between the minimum number of net positive charges (pₘ) and the total number of amino acid residues (r) is as follows:

**TABLE 2. Amino acid number and net positive charges (2pₘ ≤ p+1)**

| **(r)** | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|
| (**pₘ**) | 2 | 2 | 3 | 3 | 4 | 4 | 5 | 5 | 6 | 6 | 7 | 7 | 8 | 8 | 9 | 9 | 10 | 10 |

In one embodiment, the minimum number of net positive charges (pₘ) and the total number of amino acid residues (r) are equal. In another embodiment, the peptides have three or four amino acid residues and a minimum of one net positive charge, suitably, a minimum of two net positive charges and more preferably a minimum of three net positive charges.

It is also important that the aromatic-cationic peptides have a minimum number of aromatic groups in comparison to the total number of net positive charges (pₜ). The minimum number of aromatic groups will be referred to below as (a). Naturally occurring amino acids that have an aromatic group include the amino acids histidine, tryptophan, tyrosine, and phenylalanine. For example, the hexapeptide Lys-Gln-Tyr-D-Arg-Phe-Trp has a net positive charge of two (contributed by the lysine and arginine residues) and three aromatic groups (contributed by tyrosine, phenylalanine and tryptophan residues).

The aromatic-cationic peptides should also have a relationship between the minimum number of aromatic groups (a) and the total number of net positive charges at physiological pH (pₜ) wherein 3a is the largest number that is less than or equal to pₜ + 1, except that when pₜ is 1, a may also be 1. In this embodiment, the relationship between the minimum number of aromatic groups (a) and the total number of net positive charges (pₜ) is as follows:

**TABLE 3. Aromatic groups and net positive charges (3a ≤ pₜ+1 or a= pₜ=1)**

| **(pₜ)** | 1 | 2 | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|---|---|
| **(a)** | 1 | 1 | 1 | 1 | 2 | 2 | 2 | 3 | 3 | 3 | 4 | 4 | 4 | 5 | 5 | 5 | 6 | 6 | 6 | 7 |

In another embodiment, the aromatic-cationic peptides have a relationship between the minimum number of aromatic groups (a) and the total number of net positive charges (pₜ) wherein 2a is the largest number that is less than or equal to pₜ + 1. In this embodiment, the relationship between the minimum number of aromatic amino acid residues (a) and the total number of net positive charges (pₜ) is as follows:

**TABLE 4. Aromatic groups and net positive charges (2a ≤ pₜ+1 or a= pₜ=1)**

| **(pₜ)** | 1 | 2 | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|---|---|
| **(a)** | 1 | 1 | 2 | 2 | 3 | 3 | 4 | 4 | 5 | 5 | 6 | 6 | 7 | 7 | 8 | 8 | 9 | 9 | 10 | 10 |

In another embodiment, the number of aromatic groups (a) and the total number of net positive charges (pₜ) are equal.

Carboxyl groups, especially the terminal carboxyl group of a C-terminal amino acid, are suitably amidated with, for example, ammonia to form the C-terminal amide. Alternatively, the terminal carboxyl group of the C-terminal amino acid may be amidated with any primary or secondary amine. The primary or secondary amine may, for example, be an alkyl, especially a branched or unbranched C₁-C₄ alkyl, or an aryl amine. Accordingly, the amino acid at the C-terminus of the peptide may be converted to an amido, N-methylamido, N-ethylamido, N,N-dimethylamido, N,N-diethylamido, N-methyl-N-ethylamido, N-phenylamido or N-phenyl-N-ethylamido group. The free carboxylate groups of the asparagine, glutamine, aspartic acid, and glutamic acid residues not occurring at the C-terminus of the aromatic-cationic peptides may also be amidated wherever they occur within the peptide. The amidation at these internal positions may be with ammonia or any of the primary or secondary amines described above.

In one embodiment, the aromatic-cationic peptide is a tripeptide having two net positive charges and at least one aromatic amino acid. In a particular embodiment, the aromatic-cationic peptide is a tripeptide having two net positive charges and two aromatic amino acids.

Aromatic-cationic peptides include, but are not limited to, the following illustrative peptides:
D-Arg-Dmt-Lys-Trp-NH₂;
D-Arg-Trp-Lys-Trp-NH₂;
D-Arg-Dmt-Lys-Phe-Met-NH₂;
H-D-Arg-Dmt-Lys(*N^{α}*Me)-Phe-NH₂;
H-D-Arg-Dmt-Lys-Phe(*N*Me)-NH₂;
H-D-Arg-Dmt-Lys(*N^{α}*Me)-Phe(*N*Me)-NH₂;
H-D-Arg(*N^{α}*Me)-Dmt(*N*Me)-Lys(*N^{α}*Me)-Phe(*N*Me)-NH₂;
D-Arg-Dmt-Lys-Phe-Lys-Trp-NH₂;
D-Arg-Dmt-Lys-Dmt-Lys-Trp-NH₂;
D-Arg-Dmt-Lys-Phe-Lys-Met-NH₂;
D-Arg-Dmt-Lys-Dmt-Lys-Met-NH₂;
H-D-Arg-Dmt-Lys-Phe-Sar-Gly-Cys-NH₂;
H-D-Arg-Ψ[CH₂-NH]Dmt-Lys-Phe-NH₂;
H-D-Arg-Dmt-Ψ[CH₂-NH]Lys-Phe-NH₂;
H-D-Arg-Dmt-LysΨ[CH₂-NH]Phe-NH₂; and
H-D-Arg-Dmt-Ψ[CH₂-NH]Lys-Ψ[CH₂-NH]Phe-NH₂.

In one embodiment, the peptides have mu-opioid receptor agonist activity (*i.e.,* they activate the mu-opioid receptor). Mu-opioid activity can be assessed by radioligand binding to cloned mu-opioid receptors or by bioassays using the guinea pig ileum (Schiller et al., Eur J Med Chem, 35:895-901, 2000; Zhao et al., J Pharmacol Exp Ther, 307:947-954, 2003). Activation of the mu-opioid receptor typically elicits an analgesic effect. In certain instances, an aromatic-cationic peptide having mu-opioid receptor agonist activity is preferred. For example, during short-term treatment, such as in an acute disease or condition, it may be beneficial to use an aromatic-cationic peptide that activates the mu-opioid receptor. Such acute diseases and conditions are often associated with moderate or severe pain. In these instances, the analgesic effect of the aromatic-cationic peptide may be beneficial in the treatment regimen of the human patient or other mammal. An aromatic-cationic peptide which does not activate the mu-opioid receptor, however, may also be used with or without an analgesic, according to clinical requirements.

Alternatively, in other instances, an aromatic-cationic peptide that does not have mu-opioid receptor agonist activity is preferred. For example, during long-term treatment, such as in a chronic disease state or condition, the use of an aromatic-cationic peptide that activates the mu-opioid receptor may be contraindicated. In these instances, the potentially adverse or addictive effects of the aromatic-cationic peptide may preclude the use of an aromatic-cationic peptide that activates the mu-opioid receptor in the treatment regimen of a human patient or other mammal. Potential adverse effects may include sedation, constipation and respiratory depression. In such instances an aromatic-cationic peptide that does not activate the mu-opioid receptor may be an appropriate treatment.

Peptides which have mu-opioid receptor agonist activity are typically those peptides which have a tyrosine residue or a tyrosine derivative at the N-terminus (*i.e.,* the first amino acid position). Suitable derivatives of tyrosine include 2'-methyltyrosine (Mmt); 2',6'-dimethyltyrosine (2'6'-Dmt); 3',5'-dimethyltyrosine (3'5'Dmt); N,2',6'-trimethyltyrosine (Tmt); and 2'-hydroxy-6'-methyltryosine (Hmt).

Peptides that do not have mu-opioid receptor agonist activity generally do not have a tyrosine residue or a derivative of tyrosine at the N-terminus (*i.e.,* amino acid position 1). The amino acid at the N-terminus can be any naturally occurring or non-naturally occurring amino acid other than tyrosine. In one embodiment, the amino acid at the N-terminus is phenylalanine or its derivative. Exemplary derivatives of phenylalanine include 2'-methylphenylalanine (Mmp), 2',6'-dimethylphenylalanine (2',6'-Dmp), N,2',6'-trimethylphenylalanine (Tmp), and 2'-hydroxy-6'-methylphenylalanine (Hmp).

The peptides mentioned herein and their derivatives can further include functional analogs. A peptide is considered a functional analog if the analog has the same function as the stated peptide. The analog may, for example, be a substitution variant of a peptide, wherein one or more amino acids are substituted by another amino acid. Suitable substitution variants of the peptides include conservative amino acid substitutions. Amino acids may be grouped according to their physicochemical characteristics as follows:
(a) Non-polar amino acids: Ala(A) Ser(S) Thr(T) Pro(P) Gly(G) Cys (C);
(b) Acidic amino acids: Asn(N) Asp(D) Glu(E) Gln(Q);
(c) Basic amino acids: His(H) Arg(R) Lys(K);
(d) Hydrophobic amino acids: Met(M) Leu(L) Ile(I) Val(V); and
(e) Aromatic amino acids: Phe(F) Tyr(Y) Trp(W) His (H).

Substitutions of an amino acid in a peptide by another amino acid in the same group is referred to as a conservative substitution and may preserve the physicochemical characteristics of the original peptide. In contrast, substitutions of an amino acid in a peptide by another amino acid in a different group is generally more likely to alter the characteristics of the original peptide.

The peptides may be synthesized by any of the methods well known in the art. Suitable methods for chemically synthesizing the protein include, for example, those described by Stuart and Young in Solid Phase Peptide Synthesis, Second Edition, Pierce Chemical Company (1984), and in Methods Enzymol., 289, Academic Press, Inc, New York (1997).

### Prophylactic and Therapeutic Uses of Aromatic-Cationic Peptides.

The aromatic-cationic peptides described herein are useful to prevent or treat disease. Specifically, the disclosure provides for both prophylactic and therapeutic methods of treating a subject at risk of (or susceptible to) disease by administering the aromatic-cationic peptides described herein. Accordingly, the present methods provide for the prevention and/or treatment of disease in a subject by administering an effective amount of an aromatic-cationic peptide to a subject in need thereof.

*Oxidative Damage*. The peptides described above are useful in reducing oxidative damage in a mammal in need thereof. Mammals in need of reducing oxidative damage are those mammals suffering from a disease, condition or treatment associated with oxidative damage. Typically, the oxidative damage is caused by free radicals, such as reactive oxygen species (ROS) and/or reactive nitrogen species (RNS). Examples of ROS and RNS include hydroxyl radical, superoxide anion radical, nitric oxide, hydrogen, hypochlorous acid (HOCl) and peroxynitrite anion. Oxidative damage is considered to be "reduced" if the amount of oxidative damage in a mammal, a removed organ, or a cell is decreased after administration of an effective amount of the aromatic cationic peptides described above. Typically, the oxidative damage is considered to be reduced if the oxidative damage is decreased by at least about 10%, at least about 25%, at least about 50%, at least about 75%, or at least about 90%, compared to a control subject not treated with the peptide.

In some embodiments, a mammal to be treated can be a mammal with a disease or condition associated with oxidative damage. The oxidative damage can occur in any cell, tissue or organ of the mammal. In humans, oxidative stress is involved in many diseases. Examples include atherosclerosis, Parkinson's disease, heart failure, myocardial infarction, Alzheimer's disease, schizophrenia, bipolar disorder, fragile X syndrome and chronic fatigue syndrome.

In one embodiment, a mammal may be undergoing a treatment associated with oxidative damage. For example, the mammal may be undergoing reperfusion. Reperfusion refers to the restoration of blood flow to any organ or tissue in which the flow of blood is decreased or blocked. The restoration of blood flow during reperfusion leads to respiratory burst and formation of free radicals.

In one embodiment, the mammal may have decreased or blocked blood flow due to hypoxia or ischemia. The loss or severe reduction in blood supply during hypoxia or ischemia may, for example, be due to thromboembolic stroke, coronary atherosclerosis, or peripheral vascular disease. Numerous organs and tissues are subject to ischemia or hypoxia. Examples of such organs include brain, heart, kidney, intestine and prostate. The tissue affected is typically muscle, such as cardiac, skeletal, or smooth muscle. For instance, cardiac muscle ischemia or hypoxia is commonly caused by atherosclerotic or thrombotic blockages which lead to the reduction or loss of oxygen delivery to the cardiac tissues by the cardiac arterial and capillary blood supply. Such cardiac ischemia or hypoxia may cause pain and necrosis of the affected cardiac muscle, and ultimately may lead to cardiac failure.

The methods can also be used in reducing oxidative damage associated with any neurodegenerative disease or condition. The neurodegenerative disease can affect any cell, tissue or organ of the central and peripheral nervous system. Examples of such cells, tissues and organs include, the brain, spinal cord, neurons, ganglia, Schwann cells, astrocytes, oligodendrocytes and microglia. The neurodegenerative condition can be an acute condition, such as a stroke or a traumatic brain or spinal cord injury. In another embodiment, the neurodegenerative disease or condition can be a chronic neurodegenerative condition. In a chronic neurodegenerative condition, the free radicals can, for example, cause damage to a protein. An example of such a protein is amyloid β-protein. Examples of chronic neurodegenerative diseases associated with damage by free radicals include Parkinson's disease, Alzheimer's disease, Huntington's disease and Amyotrophic Lateral Sclerosis (also known as Lou Gherig's disease).

Other conditions which can be treated include preeclampsia, diabetes, and symptoms of and conditions associated with aging, such as macular degeneration, wrinkles.

*Mitochondrial Permeability Transitioning.* The peptides described above are useful in treating any disease or condition that is associated with mitochondria permeability transitioning (MPT). Such diseases and conditions include, but are not limited to, ischemia and/or reperfusion of a tissue or organ, hypoxia and any of a number of neurodegenerative diseases. Mammals in need of inhibiting or preventing of MPT are those mammals suffering from these diseases or conditions.

*Determination of the Biological Effect of the Aromatic-Cationic Peptide-Based Therapeutic.* In various embodiments, suitable *in vitro* or *in vivo* assays are performed to determine the effect of a specific aromatic-cationic peptide-based therapeutic and whether its administration is indicated for treatment. In various embodiments, *in vitro* assays can be performed with representative animal models, to determine if a given aromatic-cationic peptide-based therapeutic exerts the desired effect in preventing or treating disease. Compounds for use in therapy can be tested in suitable animal model systems including, but not limited to rats, mice, chicken, pigs, cows, monkeys, rabbits, and the like, prior to testing in human subjects. Similarly, for *in vivo* testing, any of the animal model systems known in the art can be used prior to administration to human subjects.

*Prophylactic Methods.* In one aspect, the invention provides a method for preventing, in a subject, disease by administering to the subject an aromatic-cationic peptide that prevents the initiation or progression of the condition. In prophylactic applications, pharmaceutical compositions or medicaments of aromatic-cationic peptides are administered to a subject susceptible to, or otherwise at risk of a disease or condition in an amount sufficient to eliminate or reduce the risk, lessen the severity, or delay the outset of the disease, including biochemical, histologic and/or behavioral symptoms of the disease, its complications and intermediate pathological phenotypes presenting during development of the disease. Administration of a prophylactic aromatic-cationic can occur prior to the manifestation of symptoms characteristic of the aberrancy, such that a disease or disorder is prevented or, alternatively, delayed in its progression. The appropriate compound can be determined based on screening assays described above.

*Therapeutic Methods.* Another aspect of the technology includes methods of treating disease in a subject for therapeutic purposes. In therapeutic applications, compositions or medicaments are administered to a subject suspected of, or already suffering from such a disease in an amount sufficient to cure, or at least partially arrest, the symptoms of the disease, including its complications and intermediate pathological phenotypes in development of the disease.

### Modes of Administration and Effective Dosages

Any method known to those in the art for contacting a cell, organ or tissue with a peptide may be employed. Suitable methods include *in vitro, ex vivo, or in vivo* methods. *In vivo* methods typically include the administration of an aromatic-cationic peptide, such as those described above, to a mammal, suitably a human. When used *in vivo* for therapy, the aromatic-cationic peptides are administered to the subject in effective amounts (*i.e.,* amounts that have desired therapeutic effect). The dose and dosage regimen will depend upon the degree of the injury in the subject, the characteristics of the particular aromatic-cationic peptide used, *e.g.,* its therapeutic index, the subject, and the subject's history.

The effective amount may be determined during pre-clinical trials and clinical trials by methods familiar to physicians and clinicians. An effective amount of a peptide useful in the methods may be administered to a mammal in need thereof by any of a number of well-known methods for administering pharmaceutical compounds. The peptide may be administered systemically or locally.

The peptide may be formulated as a pharmaceutically acceptable salt. The term "pharmaceutically acceptable salt" means a salt prepared from a base or an acid which is acceptable for administration to a patient, such as a mammal (e.g., salts having acceptable mammalian safety for a given dosage regime). However, it is understood that the salts are not required to be pharmaceutically acceptable salts, such as salts of intermediate compounds that are not intended for administration to a patient. Pharmaceutically acceptable salts can be derived from pharmaceutically acceptable inorganic or organic bases and from pharmaceutically acceptable inorganic or organic acids. In addition, when a peptide contains both a basic moiety, such as an amine, pyridine or imidazole, and an acidic moiety such as a carboxylic acid or tetrazole, zwitterions may be formed and are included within the term "salt" as used herein. Salts derived from pharmaceutically acceptable inorganic bases include ammonium, calcium, copper, ferric, ferrous, lithium, magnesium, manganic, manganous, potassium, sodium, and zinc salts, and the like. Salts derived from pharmaceutically acceptable organic bases include salts of primary, secondary and tertiary amines, including substituted amines, cyclic amines, naturally-occurring amines and the like, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperadine, polyamine resins, procaine, purines, theobromine, triethylamine, trimethylamine, tripropylamine, tromethamine and the like. Salts derived from pharmaceutically acceptable inorganic acids include salts of boric, carbonic, hydrohalic (hydrobromic, hydrochloric, hydrofluoric or hydroiodic), nitric, phosphoric, sulfamic and sulfuric acids. Salts derived from pharmaceutically acceptable organic acids include salts of aliphatic hydroxyl acids *(e.g.,* citric, gluconic, glycolic, lactic, lactobionic, malic, and tartaric acids), aliphatic monocarboxylic acids *(e.g.,* acetic, butyric, formic, propionic and trifluoroacetic acids), amino acids *(e.g.,* aspartic and glutamic acids), aromatic carboxylic acids *(e.g.,* benzoic, p-chlorobenzoic, diphenylacetic, gentisic, hippuric, and triphenylacetic acids), aromatic hydroxyl acids (e.g., o-hydroxybenzoic, p-hydroxybenzoic, 1-hydroxynaphthalene-2-carboxylic and 3-hydroxynaphthalene-2-carboxylic acids), ascorbic, dicarboxylic acids (e.g., fumaric, maleic, oxalic and succinic acids), glucoronic, mandelic, mucic, nicotinic, orotic, pamoic, pantothenic, sulfonic acids (e.g., benzenesulfonic, camphosulfonic, edisylic, ethanesulfonic, isethionic, methanesulfonic, naphthalenesulfonic, naphthalene-1,5-disulfonic, naphthalene-2,6-disulfonic and p-toluenesulfonic acids), xinafoic acid, and the like.

The aromatic-cationic peptides described herein can be incorporated into pharmaceutical compositions for administration, singly or in combination, to a subject for the treatment or prevention of a disorder described herein. Such compositions typically include the active agent and a pharmaceutically acceptable carrier. As used herein the term "pharmaceutically acceptable carrier" includes saline, solvents, dispersion media, coatings, antibacterial and antifungal agents, isotonic and absorption delaying agents, and the like, compatible with pharmaceutical administration. Supplementary active compounds can also be incorporated into the compositions.

Pharmaceutical compositions are typically formulated to be compatible with its intended route of administration. Examples of routes of administration include parenteral (e.g., intravenous, intradermal, intraperitoneal or subcutaneous), oral, inhalation, transdermal (topical), intraocular, iontophoretic, and transmucosal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic. For convenience of the patient or treating physician, the dosing formulation can be provided in a kit containing all necessary equipment (e.g., vials of drug, vials of diluent, syringes and needles) for a treatment course *(e.g.,* 7 days of treatment).

Pharmaceutical compositions suitable for injectable use can include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, a composition for parenteral administration must be sterile and should be fluid to the extent that easy syringability exists. It should be stable under the conditions of manufacture and storage and must be preserved against the contaminating action of microorganisms such as bacteria and fungi.

The aromatic-cationic peptide compositions can include a carrier, which can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thiomerasol, and the like. Glutathione and other antioxidants can be included to prevent oxidation. In many cases, it will be preferable to include isotonic agents, for example, sugars, polyalcohols such as mannitol, sorbitol, or sodium chloride in the composition. Prolonged absorption of the injectable compositions can be brought about by including in the composition an agent which delays absorption, for example, aluminum monostearate or gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle, which contains a basic dispersion medium and the required other ingredients from those enumerated above. In the case of sterile powders for the preparation of sterile injectable solutions, typical methods of preparation include vacuum drying and freeze drying, which can yield a powder of the active ingredient plus any additional desired ingredient from a previously sterile-filtered solution thereof.

Oral compositions generally include an inert diluent or an edible carrier. For the purpose of oral therapeutic administration, the active compound can be incorporated with excipients and used in the form of tablets, troches, or capsules, *e.g*., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds can be delivered in the form of an aerosol spray from a pressurized container or dispenser which contains a suitable propellant, *e.g.,* a gas such as carbon dioxide, or a nebulizer. Such methods include those described in U.S. Pat. No. 6,468,798.

Systemic administration of a therapeutic compound as described herein can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays. For transdermal administration, the active compounds are formulated into ointments, salves, gels, or creams as generally known in the art. In one embodiment, transdermal administration may be performed my iontophoresis.

A therapeutic protein or peptide can be formulated in a carrier system. The carrier can be a colloidal system. The colloidal system can be a liposome, a phospholipid bilayer vehicle. In one embodiment, the therapeutic peptide is encapsulated in a liposome while maintaining peptide integrity. As one skilled in the art would appreciate, there are a variety of methods to prepare liposomes. *(See* Lichtenberg et al., Methods Biochem. Anal., 33:337-462 (1988); Anselem et al., Liposome Technology, CRC Press (1993)). Liposomal formulations can delay clearance and increase cellular uptake (*See* Reddy, Ann. Pharmacother., 34(7-8):915-923 (2000)). An active agent can also be loaded into a particle prepared from pharmaceutically acceptable ingredients including, but not limited to, soluble, insoluble, permeable, impermeable, biodegradable or gastroretentive polymers or liposomes. Such particles include, but are not limited to, nanoparticles, biodegradable nanoparticles, microparticles, biodegradable microparticles, nanospheres, biodegradable nanospheres, microspheres, biodegradable microspheres, capsules, emulsions, liposomes, micelles and viral vector systems.

The carrier can also be a polymer, *e.g.,* a biodegradable, biocompatible polymer matrix. In one embodiment, the therapeutic peptide can be embedded in the polymer matrix, while maintaining protein integrity. The polymer may be natural, such as polypeptides, proteins or polysaccharides, or synthetic, such as poly α-hydroxy acids. Examples include carriers made of, *e.g.,* collagen, fibronectin, elastin, cellulose acetate, cellulose nitrate, polysaccharide, fibrin, gelatin, and combinations thereof. In one embodiment, the polymer is poly-lactic acid (PLA) or copoly lactic/glycolic acid (PGLA). The polymeric matrices can be prepared and isolated in a variety of forms and sizes, including microspheres and nanospheres. Polymer formulations can lead to prolonged duration of therapeutic effect. (*See* Reddy, Ann. Pharmacother., 34(7-8):915-923 (2000)). A polymer formulation for human growth hormone (hGH) has been used in clinical trials. (*See* Kozarich and Rich, Chemical Biology, 2:548-552 (1998)).

Examples of polymer microsphere sustained release formulations are described in PCT publication WO 99/15154 (Tracy et al.*),* U.S. Pat. Nos. 5,674,534 and 5,716,644 (both to Zale et al.), PCT publication WO 96/40073 (Zale et al.)*,* and PCT publication WO 00/38651 (Shah et al.)*.* U.S. Pat. Nos. 5,674,534 and 5,716,644 and PCT publication WO 96/40073 describe a polymeric matrix containing particles of erythropoietin that are stabilized against aggregation with a salt.

In some embodiments, the therapeutic compounds are prepared with carriers that will protect the therapeutic compounds against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylacetic acid. Such formulations can be prepared using known techniques. The materials can also be obtained commercially, *e.g*., from Alza Corporation and Nova Pharmaceuticals, Inc. Liposomal suspensions (including liposomes targeted to specific cells with monoclonal antibodies to cell-specific antigens) can also be used as pharmaceutically acceptable carriers. These can be prepared according to methods known to those skilled in the art, for example, as described in U.S. Pat. No. 4,522,811.

The therapeutic compounds can also be formulated to enhance intracellular delivery. For example, liposomal delivery systems are known in the art, see, *e.g.,* Chonn and Cullis, "Recent Advances in Liposome Drug Delivery Systems," Current Opinion in Biotechnology 6:698-708 (1995); Weiner, "Liposomes for Protein Delivery: Selecting Manufacture and Development Processes," Immunomethods, 4(3):201-9 (1994); and Gregoriadis, "Engineering Liposomes for Drug Delivery: Progress and Problems," Trends Biotechnol., 13(12):527-37 (1995). Mizguchi et al., Cancer Lett., 100:63-69 (1996), describes the use of fusogenic liposomes to deliver a protein to cells both *in vivo* and *in vitro.*

Dosage, toxicity and therapeutic efficacy of the therapeutic agents can be determined by standard pharmaceutical procedures in cell cultures or experimental animals, *e.g.,* for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit high therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the methods, the therapeutically effective dose can be estimated initially from cell culture assays. A dose can be formulated in animal models to achieve a circulating plasma concentration range that includes the IC50 (*i.e.,* the concentration of the test compound which achieves a half-maximal inhibition of symptoms) as determined in cell culture. Such information can be used to more accurately determine useful doses in humans. Levels in plasma may be measured, for example, by high performance liquid chromatography.

Typically, an effective amount of the aromatic-cationic peptides, sufficient for achieving a therapeutic or prophylactic effect, range from about 0.000001 mg per kilogram body weight per day to about 10,000 mg per kilogram body weight per day. Suitably, the dosage ranges are from about 0.0001 mg per kilogram body weight per day to about 100 mg per kilogram body weight per day. For example dosages can be 1 mg/kg body weight or 10 mg/kg body weight every day, every two days or every three days or within the range of 1-10 mg/kg every week, every two weeks or every three weeks. In one embodiment, a single dosage of peptide ranges from 0.1-10,000 micrograms per kg body weight. In one embodiment, aromatic-cationic peptide concentrations in a carrier range from 0.2 to 2000 micrograms per delivered milliliter. An exemplary treatment regime entails administration once per day or once a week. In therapeutic applications, a relatively high dosage at relatively short intervals is sometimes required until progression of the disease is reduced or terminated, and preferably until the subject shows partial or complete amelioration of symptoms of disease. Thereafter, the patient can be administered a prophylactic regime.

In some embodiments, a therapeutically effective amount of an aromatic-cationic peptide may be defined as a concentration of peptide at the target tissue of 10⁻¹² to 10⁻⁶ molar, *e.g*., approximately 10⁻⁷ molar. This concentration may be delivered by systemic doses of 0.01 to 100 mg/kg or equivalent dose by body surface area. The schedule of doses would be optimized to maintain the therapeutic concentration at the target tissue, most preferably by single daily or weekly administration, but also including continuous administration (e.g., parenteral infusion or transdermal application).

In some embodiments, the dosage of the aromatic-cationic peptide is provided at about 0.001 to about 0.5 mg/kg/h, suitably from about 0.01 to about 0.1 mg/kg/h. In one embodiment, the is provided from about 0.1 to about 1.0 mg/kg/h, suitably from about 0.1 to about 0.5 mg/kg/h. In one embodiment, the dose is provided from about 0.5 to about 10 mg/kg/h, suitably from about 0.5 to about 2 mg/kg/h.

The skilled artisan will appreciate that certain factors may influence the dosage and timing required to effectively treat a subject, including but not limited to, the severity of the disease or disorder, previous treatments, the general health and/or age of the subject, and other diseases present. Moreover, treatment of a subject with a therapeutically effective amount of the therapeutic compositions described herein can include a single treatment or a series of treatments.

The mammal treated in accordance present methods can be any mammal, including, for example, farm animals, such as sheep, pigs, cows, and horses; pet animals, such as dogs and cats; laboratory animals, such as rats, mice and rabbits. In a preferred embodiment, the mammal is a human.

### EXAMPLES

The present invention is further illustrated by the following examples, which should not be construed as limiting in any way.

Overexpression of catalase targeted to mitochondria (mCAT) has been shown to improve aging and prolong lifespan in mice. These examples identify "druggable" chemical compounds that can reduce mitochondrial oxidative stress and protect mitochondrial function. As mitochondria are the major source of intracellular reactive oxygen species (ROS), the antioxidant must be delivered to mitochondria in order limit oxidative damage to mitochondrial DNA, proteins of the electron transport chain (ETC), and the mitochondrial lipid membranes. We discovered a family of synthetic aromatic-cationic tetrapeptides that selectively target and concentrate in the inner mitochondrial membrane (IMM). Some of these peptides contain redox-active amino acids that can undergo one-electron oxidation and behave as mitochondria-targeted antioxidants. In particular, the peptide D-Arg-2'6'-Dmt-Tyr-Lys-Phe-NH₂ reduces mitochondrial ROS and protect mitochondrial function in cellular and animal studies. Recent studies show that this peptide can confer protection against mitochondrial oxidative stress comparable to that observed with mitochondrial catalase overexpression. Although radical scavenging is the most commonly used approach to reduce oxidative stress, there are other potential mechanisms that can be used, including facilitation of electron transfer to reduce electron leak and improved mitochondrial reduction potential.

Abundant circumstantial evidence indicates that oxidative stress contributes to many consequences of normal aging and several major diseases, including cardiovascular diseases, diabetes, neurodegenerative diseases, and cancer. Oxidative stress is generally defined as an imbalance of prooxidants and antioxidants. However, despite a wealth of scientific evidence to support increased oxidative tissue damage, large-scale clinical studies with antioxidants have not demonstrated significant health benefits in these diseases. One of the reasons may be due to the inability of the available antioxidants to reach the site of prooxidant production.

The mitochondrial electron transport chain (ETC) is the primary intracellular producer of ROS, and mitochondria themselves are most vulnerable to oxidative stress. Protecting mitochondrial function would therefore be a prerequisite to preventing cell death caused by mitochondrial oxidative stress. The benefits of overexpressing catalase targeted to mitochondria (mCAT), but not peroxisomes (pCAT), provided proof-of-concept that mitochondria-targeted antioxidants would be necessary to overcome the detrimental effects of aging. However, adequate delivery of chemical antioxidants to the IMM remains a challenge.

One peptide analog, D-Arg-2'6'-Dmt-Tyr-Lys-Phe-NH₂, possesses intrinsic antioxidant ability because the modified tyrosine residue is redox-active and can undergo one-electron oxidation. We have shown that this peptide can neutralize H₂O₂, hydroxyl radical, and peroxynitrite, and inhibit lipid peroxidation. The peptide has demonstrated remarkable efficacy in animal models of ischemia-reperfusion injury, neurodegenerative diseases, and metabolic syndrome.

The design of the mitochondria-targeted peptides incorporates and enhances one or more of the following modes of action: (i) scavenging excess ROS, (ii) reducing ROS production by facilitating electron transfer, or (iii) increasing mitochondrial reductive capacity. The advantage of peptide molecules is that it is possible to incorporate natural or unnatural amino acids that can serve as redox centers, facilitate electron transfer, or increase sulfydryl groups while retaining the aromatic-cationic motif required for mitochondria targeting. The proposed design strategies are supported by known electron chemistry and will be confirmed by chemical, biochemical, cell culture, and animal studies. State-of-the-art physical, chemical and molecular biology approaches will be used to screen the new analogs for mitochondrial ROS production and redox regulation, testing and validating the hypothesized molecular modes of action. The most promising analogs will be provided to the various projects for evaluation in mitochondria, cellular, and tissue models. The proposed studies represent a novel integrated approach to the design of mitochondria-targeted antioxidants that is significantly different from other efforts in the field.

### Example 1. Synthesis of Aromatic-Cationic Peptides.

Solid-phase peptide synthesis is used and all amino acids derivatives are commercially available. After completion of peptide assembly, peptides are cleaved from the resin in the usual manner. Crude peptides are purified by preparative reversed-phase chromatography. The structural identity of the peptides is confirmed by FAB mass spectrometry and their purity is assessed by analytical reversed-phase HPLC and by thin-layer chromatography in three different systems. Purity of >98% will be achieved. Typically, a synthetic run using 5 g of resin yields about 2.0-2.3 g of pure peptides.

### Example 2. Determination of Dosing Regimens.

The peptides are soluble in water, and it is possible to administer them parenterally (*iv, sc, ip*)*.* Pharmacokinetic studies have shown that absorption is very fast and complete after *sc* administration, and *in vivo* efficacy studies support once a day dosing for most indications. We have also determined that these peptides are stable in solution for more than 3 months at 37°C. This makes it possible to deliver these peptides via implantable miniosmotic Alzet pumps for 4 or 6 weeks to avoid daily injections. The feasibility of this route of administration has been confirmed. Our experience with long-term administration of aromatic cationic peptides in rats and mice revealed that effective doses range from 0.00 to 3 mg/kg/d, depending on the disease model. Toxicology studies have shown that the safety margin for certain aromatic-cationic peptides is very wide, and no adverse effects were observed with doses up to 300 mg/kg/d for 28 d in rats. *See, e.g.,* Stuart and Young in Solid Phase Peptide Synthesis, Second Edition, Pierce Chemical Company (1984), and in Methods Enzymol., 289, Academic Press, Inc, New York (1997).

### Example 3. Orally-Active Peptide Analogs.

Oral bioavailability of any compound is determined by water solubility, stability in gastric and intestinal fluids, and absorption across the intestinal epithelial barrier. The peptide D-Arg-2'6'-Dmt-Tyr-Lys-Phe-NH₂ is water-soluble, acid-resistant and resistant against gastric enzymes, and can be easily absorbed across the epithelial barrier. However, oral bioavailability of this peptide is limited by degradation in intestinal fluids. This Example provides new analogs that would be resistant to pancreatin activity.

One way of stabilizing peptides against enzymatic degradation is the replacement of an L-amino acid with a D-amino acid at the peptide bond undergoing cleavage. Aromatic cationic peptide analogs are prepared containing one or more D-amino acid residues in addition to the D-Arg residue already present. Another way to prevent enzymatic degradation is *N*-methylation of the α-amino group at one or more amino acid residues of the peptides. This will prevent peptide bond cleavage by *any* peptidase. Examples include: H-D-Arg-Dmt-Lys(*N^{α}*Me)-Phe-NH₂; H-D-Arg-Dmt-Lys-Phe(*N*Me)-NH₂; H-D-Arg-Dmt-Lys(*N^{α}*Me)-Phe(*N*Me)-NH₂; and H-D-Arg(*N^{α}*Me)-Dmt(*N*Me)-Lys(*N^{α}*Me)-Phe(*N*Me)-NH₂. *N^{α}*-methylated analogues have lower hydrogen bonding capacity and can be expected to have improved intestinal permeability.

An alternative way to stabilize a peptide amide bond (-CO-NH-) against enzymatic degradation is its replacement with a reduced amide bond (Ψ[CH₂-NH]). This can be achieved with a reductive alkylation reaction between a Boc-amino acid-aldehyde and the amino group of the N-terminal amino acid residue of the growing peptide chain in solid-phase peptide synthesis. The reduced peptide bond is predicted to result in improved cellular permeability because of reduced hydrogen-bonding capacity. Examples include: H-D-Arg-Ψ[CH₂-NH]Dmt-Lys-Phe-NH₂,H-D-Arg-Dmt-Ψ[CH₂-NH]Lys-Phe-NH₂,H-D-Arg-Dmt-LysΨ[CH₂-NH]Phe-NH₂, H-D-Arg-Dmt-Ψ[CH₂-NH]Lys-Ψ[CH₂-NH]Phe-NH₂, etc.

These new analogs are screened for stability in plasma, simulated gastric fluid (SGF) and simulated intestinal fluid (SIF). An amount of peptide is added to 10 ml of SGF with pepsin (Cole-Palmer) or SIF with pancreatin (Cole-Palmer), mixed and incubated for 0, 30, 60, 90 and 120 min. The samples are analyzed by HPLC following solid-phase extraction. New analogs that are stable in both SGF and SIF are then be evaluated for their distribution across the Caco-2 monolayer. Analogs with apparent permeability coefficient determined to be >10⁻⁶ cm/s (predictable of good intestinal absorption) will then have their activity in reducing mitochondrial oxidative stress determined in cell cultures. Mitochondrial ROS is quantified by FACS using MitoSox for superoxide, and HyPer-mito (a genetically encoded fluorescent indicator targeted to mitochondria for sensing H₂O₂). Mitochondrial oxidative stressors can include t-butylhydroperoxide, antimycin and angiotensin. New analogs that satisfy all these criteria can then undergo large-scale synthesis.

It is predicted that the proposed strategies will produce an analog that would have oral bioavailability. The Caco-2 model is regarded as a good predictor of intestinal absorption by the drug industry.

### Example 5. New peptide analogs with improved electron scavenging ability.

Certain natural amino acids are redox-active and can undergo one-electron oxidation, including Tyr, Trp, Cys and Met, with Tyr being the most versatile. Tyr can undergo one-electron oxidation by mechanisms that include oxidation by H₂O₂ and hydroxyl radicals. Tyrosyl radicals react poorly with O₂, but can combine to form the dityrosine dimer. Tyrosyl radicals can be scavenged by GSH to generate the thiyl radical (GS▪) and superoxide. The reaction of superoxide with phenoxyl radicals can result in either repair of the parent phenol or addition to form a hydroperoxide. The generation of the Tyr hydroperoxide is favored by certain conditions, especially if the Tyr is N-terminal or a free amine is nearby. In the existing peptides, electron scavenging has been provided by Tyr or substituted Tyr, including 2',6'-Dmt. Substitution of Tyr with Phe abolishes scavenging activity.

We predict that we can increase electron scavenging capacity of the peptides by increasing the number of redox-active amino acids. We have also found that incorporation of methyl groups on Tyr further increased the scavenging activity compared to Tyr. Furthermore, in place of Tyr, Trp or Met can be substituted into our design of aromatic-cationic peptides for mitochondria targeting. Superoxide can react with tryptophan to form a number of different reaction products, and with methionine to form methionine sulfoxide. Examples of new peptide analogs include: D-Arg-Dmt-Lys-Dmt-NH₂; D-Arg-Dmt-Lys-Trp-NH₂; D-Arg-Trp-Lys-Trp-NH₂, D-Arg-Dmt-Lys-Phe-Met-NH₂. The ability of these new analogs to scavenge H₂O₂, hydroxyl radical, superoxide, peroxynitrite, is determined *in vitro*, and then confirmed in cell cultures.

We anticipate that scavenging capacity of the peptide analogs will increase linearly with increased number of redox-active amino acids. It is important that we maintain the aromatic-cationic motif in order to retain mitochondrial targeting potential. It may be possible to increase the peptide length to 6 residues and achieve 3 times the scavenging capacity while still maintaining cell permeability.

### Example 6. New peptide analogs that facilitate electron transfer.

ATP synthesis in the ETC is driven by electron flow through the protein complexes of the ETC which can be described as a series of oxidation/reduction processes. Rapid shunting of electrons through the ETC is important for preventing short-circuiting that would lead to electron escape and generation of free radical intermediates. The rate of electron transfer (ET) between an electron donor and electron acceptor decreases exponentially with the distance between them, and superexchange ET is limited to 20Å. Long-range ET can be achieved in a multi-step electron hopping process, where the overall distance between donor and acceptor is split into a series of shorter, and therefore faster, ET steps. In the ETC, efficient ET over long distances is assisted by cofactors that are strategically localized along the IMM, including FMN, FeS clusters, and hemes. Aromatic amino acids such as Phe, Tyr and Trp can also facilitate electron transfer to heme through overlapping *π* clouds, and this was specifically shown for cyt c. Amino acids with suitable oxidation potential (Tyr, Trp, Cys, Met) can act as stepping stones by serving as intermediate electron carriers. In addition, the hydroxyl group of Tyr can lose a proton when it conveys an electron, and the presence of a basic group nearby, such as Lys, can result in proton-coupled ET which is even more efficient.

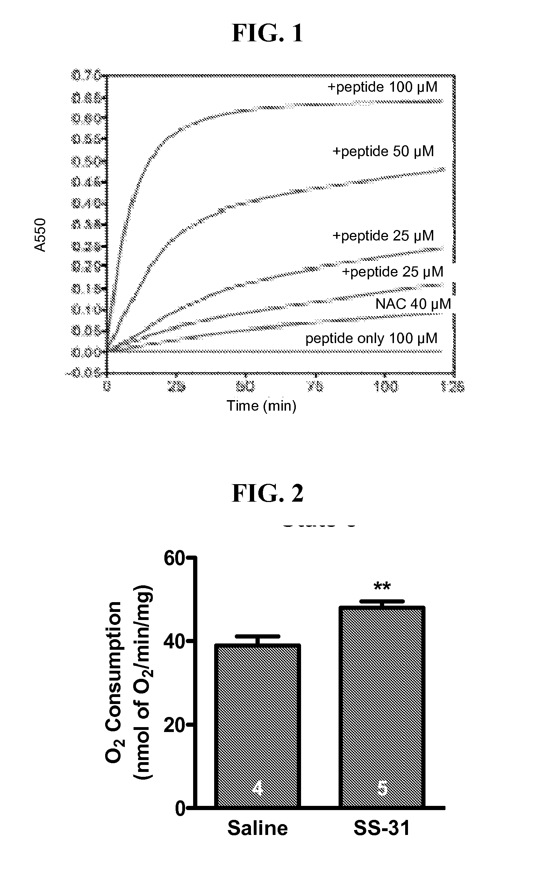

We hypothesize that the distribution of aromatic cationic peptides among the protein complexes in the IMM allows it to serve as additional relay stations to facilitate ET. In support of this hypothesis, we have used the kinetics of cyt c reduction (monitored by absorbance spectroscopy) as a simple model system to determine if the peptide D-Arg-2'6'-Dmt-Lys-Phe-NH₂ can facilitate ET. Addition of N-acetylcysteine (NAC) as a reducing agent resulted in time-dependent increase in absorbance at 550 nm (A₅₅₀) (Fig. 1). The addition of peptide alone at 100 µM concentrations did not reduce cyt c, but dose-dependently increased the rate of NAC-induced cyt c reduction, suggesting that this peptide does not donate an electron but can speed up electron transfer. Similar results were obtained with GSH as a reducing agent and the peptide H-Phe-D-Arg-Phe-Lys-NH₂.

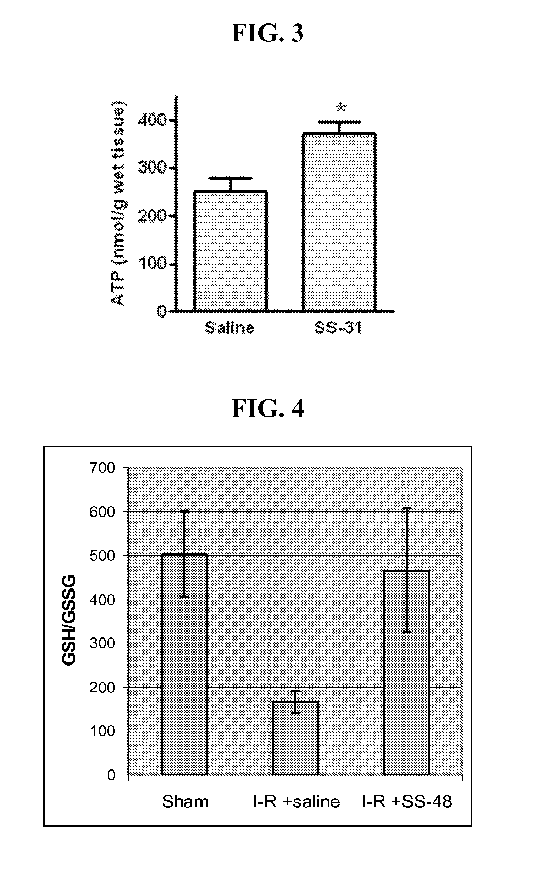

Preliminary studies further support our hypothesis that D-Arg-2'6'-Dmt-Lys-Phe-NH₂ can facilitate ET and improve ATP synthesis *in vivo.* We have examined the effect of this peptide on restoration of mitochondrial respiration and ATP synthesis following ischemia-reperfusion (IR) injury in rats. Rats were subjected to bilateral occlusion of renal artery for 45 min followed by 20 min or 1 h reperfusion. Rats received saline or peptide (2.0 mg/kg sc) 30 min before ischemia and again at the time of reperfusion (n=4-5 in each group). The results are shown in Fig. 2 and Fig. 3 and demonstrate that the peptide improved oxygen consumption and ATP synthesis.

Hexapeptide analogues are prepared, including D-Arg-Dmt-Lys-Phe-Lys-Trp-NH₂, D-Arg-Dmt-Lys-Dmt-Lys-Trp-NH₂, D-Arg-Dmt-Lys-Phe-Lys-Met-NH₂, D-Arg-Dmt-Lys-Dmt-Lys-Met-NH₂, etc. These analogs are evaluated in the cyt c reduction assay, and confirmed by electron flux assays in permeabilized muscle fibers and intact muscle, and in permeabilized cardiomyocytes and whole hearts. It is predicted that these peptides will improve oxygen consumption and ATP synthesis compared to a control.

### Example 7. New peptide analogs that can enhance mitochondrial reduction potential

The redox environment of a cell depends on its reduction potential and reducing capacity. Redox potential is highly compartmentalized within the cell, and the redox couples in the mitochondrial compartment are more reduced than in the other cell compartments and are more susceptible to oxidation. Glutathione (GSH) is present in mM concentrations in mitochondria and is considered the major redox couple. The reduced thiol group -SH can reduce disulfide S-S groups in proteins and restore function. The redox potential of the GSH/GSSG couple is dependent upon two factors: the amounts of GSH and GSSG, and the ratio between GSH and GSSG. As GSH is compartmentalized in the cell and the ratio of GSH/GSSG is regulated independently in each compartment, mitochondrial GSH (mGSH) is the primary defense against mitochondrial oxidative stress. Mitochondrial GSH redox potential becomes more oxidizing with aging, and this is primarily due to increase in GSSG content and decrease in GSH content.

The aromatic cationic peptides are used as a vector to direct the delivery of Cys into mitochondria. The -SH group of Cys in some aromatic-cationic peptides is expected to engage in a thiol-disulfide exchange reaction with GSSG to restore mitochondrial GSH/GSSG levels. Preliminary results were obtained with SS-48 (H-Phe-D-Arg-Phe-Lys-Cys-NH₂) in a rat model of renal ischemia-reperfusion (IR) injury with SS-48. Rats were subjected to bilateral occlusion of renal artery for 45 min followed by 1 h reperfusion. Rats received saline or SS-48 (0.5 mg/kg sc) 30 min before ischemia and again at the time of reperfusion (n=4 in each group). As shown in Fig. 4, SS-48 was able to maintain [GSH]/[GSSG] in IR kidneys. These results suggest that SS-48 can be used to enhance cellular uptake of Cys. Rather than a direct addition of Cys in the C terminus, we will also introduce Cys *via* a spacer, sarcosine (Sar), Sar-Gly or 7-aminoheptanoic acid. This will provide the structural flexibility at the C terminus for more efficient thiol/disulfide exchange. The following are some examples of Cys-containing analogues: H-Phe-D-Arg-Phe-Lys-Gly-Cys-NH₂, H-D-Arg-Dmt-Lys-Phe-Gly-Cys-NH₂,H-Phe-D-Arg-Phe-Lys-Sar-Cys-NH₂, and H-D-Arg-Dmt-Lys-Phe-Sar-Gly-Cys-NH₂. These new Cys-containing analogs will then be screened for their ability to improve GSH:GSSG ratio in cell cultures under oxidative stress induced by H₂O₂ or *t*BHP. Cytosolic and mitochondrial [GSH] and [GSSG] will be determined using the glutathione reductase recycling method. The successful analogs will be confirmed in heart and skeletal muscles.

### EQUIVALENTS

The present invention is not to be limited in terms of the particular embodiments described in this application, which are intended as single illustrations of individual aspects of the invention. Many modifications and variations of this invention can be made without departing from its spirit and scope, as will be apparent to those skilled in the art. Functionally equivalent methods and apparatuses within the scope of the invention, in addition to those enumerated herein, will be apparent to those skilled in the art from the foregoing descriptions. Such modifications and variations are intended to fall within the scope of the appended claims. The present invention is to be limited only by the terms of the appended claims, along with the full scope of equivalents to which such claims are entitled. It is to be understood that this invention is not limited to particular methods, reagents, compounds compositions or biological systems, which can, of course, vary. It is also to be understood that the terminology used herein is for the purpose of describing particular embodiments only, and is not intended to be limiting.

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, particularly in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, *etc.* As a nonlimiting example, each range discussed herein can be readily broken down into a lower third, middle third and upper third, *etc.* As will also be understood by one skilled in the art all language such as "up to," "at least," "greater than," "less than," and the like, include the number recited and refer to ranges which can be subsequently broken down into subranges as discussed above. Finally, as will be understood by one skilled in the art, a range includes each individual member. Thus, for example, a group having 1-3 cells refers to groups having 1, 2, or 3 cells. Similarly, a group having 1-5 cells refers to groups having 1, 2, 3, 4, or 5 cells, and so forth.

All patents, patent applications, provisional applications, and publications referred to or cited herein are incorporated by reference in their entirety, including all figures and tables, to the extent they are not inconsistent with the explicit teachings of this specification.

Other embodiments are set forth within the following claims.

## Claims

1. An aromatic cationic peptide selected from the group consisting of:
D-Arg-Dmt-Lys-Trp-NH₂;
D-Arg-Trp-Lys-Trp-NH₂;
D-Arg-Dmt-Lys-Phe-Met-NH₂;
H-D-Arg-Dmt-Lys(*N^{α}*Me)-Phe-NH₂;
H-D-Arg-Dmt-Lys-Phe(*N*Me)-NH₂;
H-D-Arg-Dmt-Lys(*N^{α}*Me)-Phe(*N*Me)-NH₂;
H-D-Arg(*N^{α}*Me)-Dmt(*N*Me)-Lys(*N^{α}*Me)-Phe(*N*Me)-NH₂;
D-Arg-Dmt-Lys-Phe-Lys-Trp-NH₂;
D-Arg-Dmt-Lys-Dmt-Lys-Trp-NH₂;
D-Arg-Dmt-Lys-Phe-Lys-Met-NH₂;
D-Arg-Dmt-Lys-Dmt-Lys-Met-NH₂;
H-D-Arg-Dmt-Lys-Phe-Sar-Gly-Cys-NH₂;
H-D-Arg-Ψ[CH₂-NH]Dmt-Lys-Phe-NH₂;
H-D-Arg-Dmt-Ψ[CH₂-NH]Lys-Phe-NH₂;
H-D-Arg-Dmt-LysΨ[CH₂-NH]Phe-NH₂; and
H-D-Arg-Dmt-Ψ[CH₂-NH]Lys-Ψ[CH₂-NH]Phe-NH₂.

2. A pharmaceutical composition comprising the aromatic cationic peptide of claim 1 and pharmaceutically acceptable salts thereof.

3. The pharmaceutical composition of claim 2 further comprising a pharmaceutically acceptable carrier.

4. A composition for use in reducing oxidative damage in a mammal in need thereof, the composition comprising a therapeutically effective amount of one or more aromatic cationic peptides of claim 1.

5. A composition for use in reducing the number of mitochondria undergoing mitochondrial permeability transitioning (MPT), or preventing mitochondrial permeability transitioning in a mammal in need thereof, the composition comprising a therapeutically effective amount of one or more aromatic cationic peptides of claim 1.
